# EUROPÄISCHE PATENTSCHRIFT

(11) **EP 3 829 425 B1**
(45) Veröffentlichungstag und Bekanntmachung des Hinweises auf die Patenterteilung: **13.08.2025**
(21) Anmeldenummer: 19756100.4
(22) Anmeldetag: 29.07.2019
(51) Int. Cl.: A61B 5/021, A61B 5/00, G01L 7/18

(54) **DRUCKSENSOREINRICHTUNG FÜR MEDIZINISCHE IN VIVO ANWENDUNG, UND HERSTELLUNGSVERFAHREN FÜR EINE SOLCHE EINRICHTUNG**
PRESSURE SENSOR DEVICE FOR MEDICAL IN VIVO APPLICATION, AND METHOD OF MANUFACTURING SUCH DEVICE
DISPOSITIF DE DÉTECTION DE PRESSION POUR APPLICATION MÉDICALE IN VIVO, ET PROCÉDÉ DE FABRICATION D'UN TEL DISPOSITIF

(30) Priorität: 27.07.2018 DE 102018005911
(43) Veröffentlichungstag der Anmeldung: 09.06.2021
(73) Patentinhaber: EMKA Medical GmbH, 63739 Aschaffenburg (DE)
(72) Erfinder: GLOCKER, Felix, 63741 Aschaffenburg (DE)
(74) Vertreter: Lüdcke, Joachim Moritz
(86) Internationale Anmeldenummer: PCT/EP2019/000229
(87) Internationale Veröffentlichungsnummer: WO 2020/020480

(56) Entgegenhaltungen:
- EP-A1- 1 514 512
- EP-A2- 2 022 396
- US-A- 4 960 411
- US-B1- 6 296 615
- US-B2- 8 142 362
- 3M: "3M Fluorinert (TM) Liquids For Electronics Manufacturing", 1 December 2003 (2003-12-01), pages 1 - 4, XP055637208, Retrieved from the Internet <URL:http://extcon.co.uk/fc74/versions.pdf> [retrieved on 20191029]
- OREN M. ROTMAN ET AL: "Method for High Accuracy Differential Pressure Measurements Using Fluid-Filled Catheters", ANNALS OF BIOMEDICAL ENGINEERING, vol. 42, no. 8, 9 May 2014 (2014-05-09), New York, pages 1705 - 1716, XP055637258, ISSN: 0090-6964, DOI: 10.1007/s10439-014-1026-4

## Beschreibung

Die Erfindung betrifft eine Drucksensoreinrichtung für medizinische in vivo Anwendung am lebenden menschlichen oder tierischen Körper, ein Messsystem und ein Herstellverfahren.

Die Bestimmung des Blutdrucks gehört zu den gängigsten und verbreitetsten Maßnahmen zur Erfassung von Vitalparametern in der medizinischen Diagnose, Therapie und Pflege. Dabei wir methodisch die direkte Blutdruckmessung von der indirekten Blutdruckmessung unterschieden.

Bei der direkten Blutdruckmessung (IBP), die man auch als invasive oder blutige Messung bezeichnet, wird ein Messfühler über einen arteriellen Zugang direkt in eine Arterie eingeführt. Alternativ wird ein Zugang zu einer Arterie gelegt und extrakorporal mit einem Messaufnehmer verbunden. Die direkte intraarterielle Methode wird wegen der damit verbundenen Infektionsrisiken und der erforderlichen Geräteinfrastruktur nur in der Intensivtherapie, auf speziell ausgestatteten Krankenstationen und im Operationssaal eingesetzt. Bei der indirekten bzw. unblutigen oder nicht-invasiven Blutdruckmessung (NIBP) verwendet man ein elektronisches Blutdruckmessgerät oder eine Blutdruckmanschette und ein Stethoskop. Die mit dieser Methode gewonnen Werte sind etwas ungenauer als die der direkten Blutdruckessung. Die indirekte Blutdruckmessung findet weitaus häufiger auf Stationen und in Arztpraxen Anwendung und kann auch außerhalb medizinischer Einrichtungen vom Patienten selbst durchgeführt werden.

Beispielsweise ist aus der WO 2009/115223 ein extrakorporal direkt messendes Blutdruckmeßsystembekannt mit einem Systemelement, das als "Druckdom" bekannt ist, was von der kuppelförmigen Ausbildung der Messkammer herrührt. Ein Messwertaufnehmer, mit dem ein solcher Dom zur Druckmessung bzw. -überwachung üblicherweise verbunden wird, ist unter der Bezeichnung "Transducer" bekannt, worunter ein Messwandler in einem geeigneten Gehäuse verstanden wird, der die üblicherweise über die Membran des Druckdomes übertragenen Drücke und Druckänderungen in ein elektrisches Signal umsetzt.

Die Membran in einem solchen Dom dient zum infektionssicheren Abschluss der blutigen Verbindung mit dem Patienten. Der Transducer ist mit einem elektronischen Diagnose- und Überwachungsgerät zur Anzeige oder Auswertung der Messsignale verbunden. Der Vorteil bei einer solchen Anordnung ist die Möglichkeit, das Systemelement als preiswertes und einfach zu entsorgendes Einwegteil auszubilden und dabei einen hygienisch einwandfreien und sicheren Abschluss des blutigen Fluidsystems sicherzustellen, siehe WO 99/37983 A2.

Extrakorporale System haben den Vorteil, dass diese einfach über einen in das das extrakorporal geführte Leitungssystem eingebundenen Dreiwegehahn durch Öffnung des Seitenarms des Dreiwegehahns auf Umgebungsdruck kalibriert werden können. Wegen der typischerweise in Intensivpflegezimmern oder Operationssälen konstanten Raumtemperatur sind darüber hinaus weitere Maßnahmen, z.B. zur Temperaturkompensation, nicht erforderlich.

Demgegenüber haben implantierte Druckmessaufnehmer den Nachteil, dass diese Temperaturschwankungen ausgesetzt sind und nicht durch Verbindung mit der Umgebung genullt werden können, d.h. die Nullpunktdrift des implantierten Drucktransducers muss sehr nahe bei Null sein, d.h. auch über lange Zeit von Monaten innerhalb der Mess-Toleranzen gemäß IEC 60601-2-34 verbleiben.

Weiter unterliegen implantierbare Druckmessaufnehmer starken Beschränkungen hinsichtlich des verfügbaren Bauraums. Für den Einsatz selbst in großen Blutgefäßen darf die Querausdehnung des Druckmessaufnehmers in der Regel nicht mehr als etwa 1 mm betragen, denn der invasive Drucktransducer darf kein Hindernis in der Blutströmung darstellen. Daher sind räumlich größere Konstruktionen für die Kompensationen der Temperaturdrift nicht realisierbar. Weiter muss die eigentliche Drucktransducer-Schaltung eine elektrische Isolationsbarriere umfassen, sowohl bei einem kapazitiven Druckmessaufnehmer mit zumindest einer beweglichen Kondensatorplatte, als auch bei einem piezoresistiven Druckmessaufnehmer mit einer Silizium-Membran mit aufgeätzter Wheatstone-Brücke, was den verfügbaren Bauraum weiter einschränkt.

Aus der DE 10 2015 116 648 A1 ist eine implantierbare Drucksensorvorrichtung mit einer Gehäuseanordnung bekannt, bei der die Gehäuseanordnung Außenwände und ein Innenvolumen aufweist und die Gehäuseanordnung zumindest zwei Druckübertragungsmembranen umfasst, die jeweils eine Oberfläche besitzen und die Oberflächen nicht in einer Ebene angeordnet sind. Die vorgeschlagnen MEMS-Chips müssen in einem reaktiven Medium, wie z.B. in Blut, geschützt werden. Dazu werden sie typischerweise in einer inkompressiblen und inerten Flüssigkeit eingebettet und in einem Gehäuse hermetisch gegen das reaktive Medium abgeschlossen. Die Flüssigkeit (z.B. Öl) dient dabei als Druckübertragungsmedium, sodass der äußere Druck über das Gehäuse zum MEMS-Chip geleitet werden kann. Als Gehäusematerial wir Titan wegen seiner Langzeitstabilität und hohen Biokompatibilität als geeignet vorgeschlagen.

Im Blutkreislauf eines Patienten können gewöhnlich Temperaturänderungen von mehreren Grad Celsius auftreten, wodurch es zu Volumenänderungen des Druckübertragungsmediums innerhalb des Gehäuses kommt. Ein weiteres Problem ist die Temperaturänderung während einer Sterilisation, beispielsweise mittels Ethylenoxid, wobei Temperaturdifferenzen von ca. 30 Grad Celsius vorkommen. Die daraus resultierende Volumen- und Druckzunahme kann bei einem konventionellen Drucksensorgehäuse die Membranen oder den MEMS-Chips beschädigen. Da das Gehäuse üblicherweise eine geringe Nachgiebigkeit bzw. Elastizität aufweist, führen die besagten Volumenänderungen des Druckübertragungsmediums zu hohen Druckschwankungen innerhalb des Gehäuses. Dieser Umstand ist zum einen durch die Materialeigenschaften des Gehäuses, zum anderen durch die zur Gehäusegröße verhältnismäßig dicken Gehäusewände bedingt. Resultierend werden die Druckmesswerte des MEMS-Chips verfälscht, da die zu messenden Druckwerte durch temperaturbedingte Druckschwankungen im Inneren des Gehäuses überlagert werden.

In US 8 573 062 B2 ist für eine MEMS-Chip Sensoranordnung die Verwendung einer Druckübertragungsmembran beschrieben, welche ein Fenster bedeckt, das in die Seitenwand des Gehäuses der Sensoranordnung eingelassen ist. In US 8 142 362 B2 ist eine an der Stirnseite des Gehäuses angeordnete Druckübertragungsmembran beschrieben.

Um eine Drucksensorvorrichtung bereitzustellen, die überwiegend unempfindlich gegenüber Temperaturänderungen in der Betriebsatmosphäre und die damit auftretenden Materialspannungen ist, wird in der DE 10 2015 116 648 A1 eine Gehäuseanordnung vorgeschlagen, die zumindest zwei Druckübertragungsmembranen aufweisen soll, die jeweils eine Oberfläche besitzen, wobei die Oberflächen nicht in einer Ebene angeordnet sind. Mit dem Einsatz von zwei Druckübertragungsmembranen werden für das Gehäuse der implantierbaren Drucksensorvorrichtung zwei nicht zusammenhängende Flächen mit größerer Nachgiebigkeit bzw. Elastizität geschaffen, sodass der Druck im Inneren des Gehäuses bei Temperaturschwankungen hinreichend reduziert wird. Indem die zwei Druckübertragungsmembranen nicht in einer Ebene angeordnet sind, können auch die durch Volumenänderung im Inneren des Gehäuses entstehenden Spannungen und Kräfte auf das Gehäusematerial sich mindestens teilweise kompensieren, wodurch die Gehäuseanordnung an Robustheit gewinnen soll.

Zur Lösung des Problems zu großer Abmessungen bei einem Druckmessaufnehmer mit integrierter Temperaturkompensation wird in der US 2004/0073122 A1 eine langgestreckte Anordnung vorgeschlagen, die an ihrer distalen Stirnseite mit einem elastischen Gelpfropfen zur Druckübertragung verschlossen ist. Der Gelpropfen soll durch einen Anwender abnehm- und austauschbar sein und aus einem vollvernetzten Mehrkomponenten-Silikon mit einem Precoursor und einem Weichmacher hergestellt werden. Für einige Ausführungsformen wird vorgeschlagen, den Gelpfropfen außenseitig mit einer zusätzlichen Membrane aus einem biokompatiblen Material abzudecken.

Aus der EP 1 514 512 A1 ist ein Katheter zur Verwendung bei der Behandlung von Hydrocephalus bekannt, der zumindest zwei Lumen aufweist, von denen eines mit einem Fluid gefüllt und hermetisch abgedichtet ist. Dieses Lumen soll zur Messung von intra-ventrikulären Drücken in Fluidverbindung mit einem Drucksensor stehen und anstelle des Gelpfropfens an dem distelen Ende ein mit einer Membran abgedecktes seitliches Loch aufweisen. Die Besonderheit soll dabei sein, dass die Membran nach Befüllen des zweiten Lumens mit dem Fluid durch Sprühbeschichten einer lösungsmittelbasierten Silikonlösung über die Öffnung zum Abdichten des Fluids in dem Katheter gebildet werden soll.

Zur Minimierung der Drift wird in der US 2011/0209553 A1 vorgeschlagen, einen piezoresistiven Druckmessaufnemer mit zwei Messkammern auszustatten, wobei eine der Messkammern hermetisch abgedichtet ist und in der Messkammer eine vorbestimmter Referenzdruck eingestellt sein soll. Durch Vergleichsmessung soll die Drift minimiert werden. Einen ähnlichen Weg schlagen Jiachou Wang and Xinxin Li in "A dual-unit pressure sensor for on-chip self-compensation of zeropoint temperature drift", Journal of Micromechanics and Microengineering, 24 (2014) 085010 (doi:10.1088/0960-1317/24/8/085010) vor.

In Rotman et al.: "Method for High Accuracy Differential Pressure Measurements Using Fluid-Filled Catheters", Annals of Biomedical Engineering, May 2014, DOI: 10.1007/s10439-014-1026-4, wird von Versuchen zur Verringerung von Messfehlern berichtet. Dazu soll der Differenzdruck zwischen zwei beiden Lumen eines Katheters gemessen werden, wobei die Lumen seitliche Öffnungen in der Nähe der Katheterspitze aufweisen, die voneinander etwa 3 cm entfernt sind. Die Messeigenschaften sollen verbessert werden durch Verwendung von destilliertem Wasser als Arbeitsfluid, das durch Erwärmung auf ca. 50°C und anschließende Behandlung in einer Vakuumkammer für eine Stunde zur Entgasung vorbehandelt wurde. Dann wurden zwei Lumen gründlich mit dem destillierten Wasser gespült und der Katheter anschließend für eine Stunde bei ca. 37°C in der aufgebauten Messschleife zur Beruhigung belassen.

In der US 2011/0040206 A1 wird eine Anordnung mit zwei Membranen vorgeschlagen, bei der eine der Membranen zur Offset-Kompensation elektrisch deformierbar sein soll.

Aus der DE 19 19 246 B2 ist eine Elektrodenanordnung zur elektrischen Reizung der rechten Herzkammer bekannt, mit einer aus einem Isolierstoffstrang bestehenden und zu einem Herzschrittmacher verlaufenden Verbindungsleitung, durch die ein elektrischer Leiter zu der auf der Außenseite des Isolierstoffstranges angeordneten Elektrode führt. Der Isolierstoffstrang soll dabei so flexibel sein, dass seine Einführung allein durch die Mitnahme durch den Blutstrom des Herzens möglich sein soll. Das soll den Nachteil haben, dass elektrische Leiter, die in den Strang aus Kunststoff in Längsrichtung nicht verschieblich eingebettet sind, bei einer Biegung des Isolierstoffstrangs stark auf Dehnung beansprucht werden, so dass sie reißen. Das soll besonders leicht vor kommen, wenn die Elektrodenanordnung etwas zurückgezogen wird, um ihre Lage zu verändern. Um die Gefahr des Reißens der Leiter bei einer solchen Elektrodenanordnung zu verhindern, wird vorgeschlagen, durch den Isolierstoffstrang eine zugfeste Seele verlaufen zu lassen. Bei Verwendung zweier elektrischer Leiter, die zur Vermeidung parasitärer Kapazitäten nicht eng benachbart in einem zentralen Kanal bei schlauchförmiger Ausführung des Stranges verlaufen sollen, ist eine Anordnung der Seele zentral zwischen den Leitern für maximale Schutzwirkung erforderlich. Die Seele soll die in dem Isolierstoffstrang auftretenden Zugkräfte aufnehmen. Durch die Anordnung zwischen den elektrischen Leitern soll eine unterschiedliche Biegefähigkeit des Isolierstoffstranges in verschiednen Biegerichtungen erreicht werden. Aus der US 4,960,411 ist ein steuerbarer Dilatationskatheter bekannt, bei dem in einem äußeren Katheter ein gegen den äußeren Katheter leicht drehbarer innerer Katheter und in dem inneren Katheter ein Steuerdraht angeordnet sein soll, der mit der Spitze des inneren Katheters fest verbunden ist. Durch Ziehen bzw. Vorschieben des Steuerdrahts wird die Spitze des inneren Katheters zur Seite bewegt. Es werden verschiedene Ausführungsformen beschrieben, bei denen entweder ein Führungsdraht oder eine Druckmessfaser zusätzlich in dem inneren Katheter angeordnet ist.

Aus der DE 000068923703 T2 und der EP 0 417 171 B1 ist eine Vorrichtung zum Messen von Körperdrücken oder physiologischen Drücken bekannt, die besonders nützlich für eine fortwährende Messung von Drücken sein soll. Dazu wird vorgeschlagen, eine Druckgeber-Kathetereinrichtung zum Übertragen des physiologischen Druckes an eine Druckwandlereinrichtung, die einen hohlen, flexiblen Schlauch aufweist mit einem ersten Ende zum Anordnen an einer Stelle, an der der physiologische Druck gemessen werden soll, und einem zweiten Ende, das mit der Druckwandlereinrichtung in Verbindung steht, und eine Flüssigkeit, die den Schlauch füllt und eine Verbindung mit der Druckwandlereinrichtung bildet, wobei ein Stöpsel am ersten Ende im Schlauch positioniert ist, wobei der Stöpsel ein Material aufweist, das in der Lage ist, Druck an die Flüssigkeit zu übertragen, die diesen Druck wiederum an die Druckwandlereinrichtung überträgt.

Aus der US 6,296,615 B1 ist eine ähnliche Vorrichtung bekannt, bei der jedoch der Katheter anstelle des Stöpsels an seinem distalen Ende mit einem Gelpfropfen verschlossen sein soll. Das Gel soll so beschaffen sein, dass es sich innerhalb des distalen Endes des Katheters infolge Druck- und Temperaturänderungen etwas bewegen kann. Der Katheter ist proximal mit einer ersten Flüssigkeit teilweise befüllt, die in Fluidkontakt mit einem Druckmessgeber steht und die den Hohlraum in dem Drucktransducer im Wesentlichen ausfüllt. Der restliche Raum zwischen erster Flüssigkeit und Gelpfropfen ist mit einer zweiten Flüssigkeit gefüllt. Die beiden Flüssigkeiten sollen unterschiedliche Eigenschaften aufweisen und untereinander nicht mischbar sein. Die erste Flüssigkeit soll dabei so gewählt werden, dass Leckage zwischen Sensor und Gehäuse minimiert wird.

Aus der EP 2 022 396 A2 ist eine implantierbare Vorrichtung zur Erfassung von intrakraniellen Drücken bekannt, wobei die Druckübertragung von außen nach innen durch eine Membrane erfolgt, deren druckabhängige Bewegung über ein Übertragungsmittel auf die Druckmesseinrichtung einwirkt. Das starre Gehäuse der Vorrichtung ist teilweise mit einer Kunststoffummantelung versehen, die die Fläche der Membran freilässt.

Der Erfindung liegt daher die Aufgabe zugrunde, eine Drucksensoreinrichtung für medizinische in vivo Anwendung bereitzustellen, die zur einfachen und sicheren Anwendung am lebenden menschlichen oder tierischen Körper geeignet und kostengünstig herstellbar ist. Die Erfindung ist bestimmt durch die Drucksensoreinrichtung gemäß den Ansprüchen 1-12, durch das Messsystem gemäß Anspruch 13, und durch das Herstellungsverfahren gemäß Anspruch 14.

Diese Aufgabe wird erfindungsgemäß gelöst durch eine Drucksensoreinrichtung der eingangs erwähnten Art, mit wenigstens einem Druckmesswandler und einer implantierbaren Sonde, wobei die Sonde proximal mit dem wenigstens einen Druckmesswandler verbunden ist, die Sonde ferner einen Katheterabschnitt und eine Messspitze am distalen Ende der Sonde umfasst, wobei die Sonde eine Längserstreckung entlang des Katheterabschnitts hat, wobei der Katheterabschnitt wenigstens ein Lumen aufweist zur Herstellung einer Fluidverbindung von der Messspitze zu dem Druckmesswandler und das Lumen mit einer Übertragungsflüssigkeit gefüllt ist, wobei die Messspitze einen rohrförmigen Abschnitt umfasst mit wenigstens einer lateral angeordneten Öffnung, wobei die wenigstens eine Öffnung durch eine elastische Membran abgedeckt ist, und wobei die Füllmenge der Übertragungsflüssigkeit so bestimmt ist, dass bei einer vorbestimmten Temperatur der Übertragungsflüssigkeit die Wölbung der Membran quer zur Längserstreckung mit der Kontur der Messspitze quer zur Längserstreckung im Wesentlichen fluchtet.

Die Aufgabe wird ferner gelöst durch ein Verfahren zur Herstellung einer Drucksensoreinrichtung für eine medizinische in vivo Anwendung mit einer implantierbaren Sonde, wobei die Sonde einen Katheterabschnitt und eine Messspitze am distalen Ende der Sonde umfasst, wobei die Sonde eine Längserstreckung entlang des Katheterabschnitts hat, wobei der Katheterabschnitt wenigstens ein Lumen aufweist zur Aufnahme einer Übertragungsflüssigkeit, wobei die Messspitze einen rohrförmigen Abschnitt umfasst mit wenigstens einer lateral angeordneten Öffnung, wobei die wenigstens eine Öffnung durch eine elastische Membran abgedeckt ist, mit folgenden Schritten: Befüllen des Lumens und der Messspitze mit der temperierten Übertragungsflüssigkeit so weit, dass die Wölbung der Membran quer zur Längserstreckung mit der Kontur der Messspitze quer zur Längserstreckung im Wesentlichen fluchtet, insbesondere nicht über die Kontur der Messspitze quer zur Längserstreckung hinausragt, Temperieren der Sonde mit der Übertragungsflüssigkeit auf eine vorbestimmte Temperatur oberhalb der für die Drucksensoreinrichtung vorgesehenen Einsatztemperatur, vorzugsweise etwa 1 K bis etwa 5 K höher als die vorgesehene Einsatztemperatur, insbesondere auf etwa 40°C bis 46°C, insbesondere um 45°C, und blasenfreies Verschließen des Lumens.

Mit einer solchen Anordnung und Verfahren lässt sich eine erfindungsgemäße Drucksensoreinrichtung so einstellen, dass die Membran bei der vorgesehenen Betriebstemperatur während der Messung möglichst spannungsfrei ist und damit eine Beeinflussung der Druckübertragung auf den Druckmesswandler minimal gering ist. Darüber hinaus ist es möglich, eine Sonde einer erfindungsgemäßen Drucksensoreinrichtung ohne Risiko einer Beschädigung durch eine nur geringfügig größere Schleuse, z.B. mit einem gegenüber der Sonde lediglich 1F größerem Innendurchmesser, zu implantieren.

Durch die erfindungsgemäße Anordnung ist es auch möglich, mit der implantierbaren Sonde den Blutdruck und insbesondere die Druckverläufe eines Patienten zu überwachen, ohne das elektronische Komponenten wie Druckmesswandler ebenfalls implantiert werden müssen. Dadurch verringert sich der bürokratische Aufwand bei Herstellung und Anwendung einer erfindungsgemäßen Drucksensoreinrichtung gegenüber bekannten implantierbaren Druckmesseinrichtungen erheblich. Weiterhin ist es mit der erfindungsgemäßen Drucksensoreinrichtung auch möglich, den Druck und die Druckverläufe bei einem Patienten zu überwachen, während dieser mit Geräten behandelt wird, die starke elektrische Störungen verursachen, z.B. einem Ablationskatheter.

Für eine zuverlässig dichte Befestigung der Membran an der Sonde ist es besonders günstig, wenn der rohrförmige Abschnitt der Messspitze von einem elastischen Schlauch umhüllt ist und die Membran aus einem ähnlichen Material besteht, wie der Schlauch, vorzugsweise beide aus einem Polyurethan hergestellt sind. Dabei weist für eine einfache Montage zweckmäßig der elastische Schlauch eine Öffnung auf, die die Öffnung in dem rohrförmigen Abschnitt überdeckt und wobei die Membran dicht mit dem Schlauch verbunden ist, wobei die Öffnung in dem Schlauch vorzugsweise geringfügig größer ist, als die Öffnung in dem rohrförmigen Abschnitt.

Die Messgenauigkeit und insbesondere die nutzbare Auflösung zur Analyse von Drucksignalen lässt sich verbessern, wenn der rohrförmige Abschnitt druckstabil ausgeführt, z.B. aus einem Metallrohr gebildet ist, vorzugsweise aus einem medizinischen rostfreien Stahl oder einer Titanlegierung.

Weiter ist es besonders vorteilhaft, wenn in dem Lumen, das die Übertragungsflüssigkeit enthält, eine flexible Seele eingebettet ist. Dadurch wird die Gefahr eines Knickens des Katheterabschnitts verringert, was zu einem Abquetschen des Lumens und damit zu einem Ausfall der Druckübertragung auf den wenigstens einen Druckmesswandler führen könnte. Durch die Seele in dem Lumen wird die Menge an Überragungsflüssigkeit verringert, was nicht nur die Herstellungskosten für eine erfindungsgemäße Drucksensoreinrichtung vermindert, sondern auch den Einfluss der thermischen Ausdehnung der Übertragungsflüssigkeit auf die Messgenauigkeit.

Selbst wenn ein Abknicken der Sonde bei beengten Platzverhältnissen erfolgen sollte, wie sie z.B. in einem Rettungswagen auftreten können, wird der Fluiddurchgang in dem Lumen nicht völlig verschlossen, da die Wandungen des Katheterabschnitts durch die abgeknickte Seele auseinandergedrückt werden.

Besonders vorteilhaft ist es dabei, wenn die Seele aus einem Polyamid besteht, vorzugsweise aus einem Polyamid 11 oder einem Polyamid 12. Eine Seele aus Polyamid 12 (PA12) bietet den besonderen Vorteil, dass eine erfindungsgemäße Druckmesseinrichtung auch bei operativen Eingriffen unter laufender Röntgenkontrolle gut eingesetzt werden kann, da bei einer solchen Ausführungsform die Seele gegen Röntgenstrahlung beständig ist. Weiter bietet eine Seele aus Polyamid 12 besondere Knickfestigkeit des Katheterabschnitts der Sonde und damit eine besonders hohe Betriebssicherheit der erfindungsgemäßen Druckmesseinrichtung bei Einsatz unter beengten Platzverhältnissen, z.B. in einem Rettungswagen, einem Ambulanzflugzeug, einem mobilen Krankenhaus oder einer mobilen Sanitätseinrichtung. Eine Seele aus Polyamid 11 (PA 11) bietet den Vorzug der Anwendbarkeit gängiger Sterilisierverfahren, ist autoklavierbar, kann mit Ethylenoxid chemisch und mittels Gamma-Strahlung strahlensterilisiert werden. Auch eine Seele aus Polyamid 11 bietet eine hohen Knickschutz für den Katheterabschnitt der Sonde. Darüber hinaus ist eine Seele aus einem Polyamid 11 als physiologisch unbedenklich einzustufen.

Für eine gute Druckübertragung über die Membran auf die Übertragungsflüssigkeit mit geringen Verfälschungen durch Spannungen innerhalb der Membran ist es zweckmäßig, wenn die wenigstens eine Öffnung in dem rohrförmigen Abschnitt eine in Richtung der Längserstreckung größere Ausdehnung aufweist, als in Umfangsrichtung, vorzugsweise das Verhältnis der Ausdehnung in Richtung der Längserstreckung zur Ausdehnung in Umfangsrichtung wenigstens 5:1 beträgt, insbesondere etwa 10:1.

Einen besonders breiten Betriebsbereich, in dem das Messergebnis praktisch frei von Beeinflussung durch Spannungen innerhalb der Membran infolge von temperaturänderungsbedingten Volumenänderungen der Übertragungsflüssigkeit ist, kann erhalten werden, wenn die wenigstens eine Öffnung in dem rohrförmigen Abschnitt in einer Richtung quer zu der Längserstreckung einen von der Membran abgedeckten Umfangsbogenabschnitt aufweist, der mit der Kontur der Messspitze quer zur Längserstreckung im Wesentlichen fluchtet, und einen Sehnenabschnitt, der die einander gegenüberliegenden Ränder der Öffnung verbindet, wobei das Verhältnis der Länge des Umfangsbogenabschnitts zur Länge der Sehne zwischen 1,33 und 1,67, bevorzugt zwischen 1,5 und 1,6, besonders bevorzugt etwa 1,57 beträgt

Als besonders vorteilhaft und betriebssicher hat sich bei Versuchen herausgestellt, wenn die Übertragungsflüssigkeit eine in Wasser unlösliche perfluorierte Flüssigkeit umfasst, wobei die perfluorierte Flüssigkeit einen Siedepunkt bei Normaldruck von wenigstens 150°C, vorzugsweise von etwa 165°C, aufweist und vollständig verdunstbar ist. Eine solche Übertragungsflüssigkeit reagiert praktisch nicht mit anderen Materialien der Drucksensoreinrichtung, gast bei den in vivo vorkommenden Körpertemperaturen nicht aus und verhält sich selbst bei einer Beschädigung während der Handhabung inert und minimiert damit die Gefährdung von Pflegepersonal und Patienten.

Gutes Druckübertragungsverhalten erhält man, wenn die Übertragungsflüssigkeit eine kinematische Viskosität von etwa 2 mm²/s bis 2,2 mm²/s bei 25°C und/oder einen Ausdehnungskoeffizienten von 0,0012 K⁻¹ und/oder eine Oberflächenspannung von etwa 16 mN/m aufweist.

Wirtschaftlich besonders gut umsetzen lässt sich die Erfindung mit einem Messsystem enthaltend wenigstens eine solche Drucksensoreinrichtung.

Die Erfindung soll nachfolgend anhand eines in den Zeichnungen dargestellten Ausführungsbeispiels näher erläutert werden. Es zeigen:
- Fig. 1: eine schematische Gesamtansicht einer erfindungsgemäßen Drucksensoreinrichtung, teilweise im Schnitt,
- Fig. 2: eine vergrößerte Darstellung auf einen rohrförmigen Abschnitts einer Messspitze der Drucksensoreinrichtung aus Fig. 1,
- Fig. 3: die schematische Gesamtansicht einer weiteren erfindungsgemäßen Drucksensoreinrichtung aus Fig. 1, teilweise im Schnitt, mit Darstellung der Anordnung einer Seele,
- Fig. 4: eine Seitenansicht des rohrförmigen Abschnitts aus Fig. 2,
- Fig. 5 bis 7: vergrößerte Querschnittansichten der Messspitze einer erfindungsgemäßen Drucksensoreinrichtung während unterschiedlicher Stadien der Befüllung mit einer Übertragungsflüssigkeit, und
- Fig. 8a bis c: eine Ansicht von unterschiedlichen Längenverhältnissen von Umfangsbogenabschnitt und Sehnenabschnitt anhand von schematischen Querschnittansichten der Messspitze.

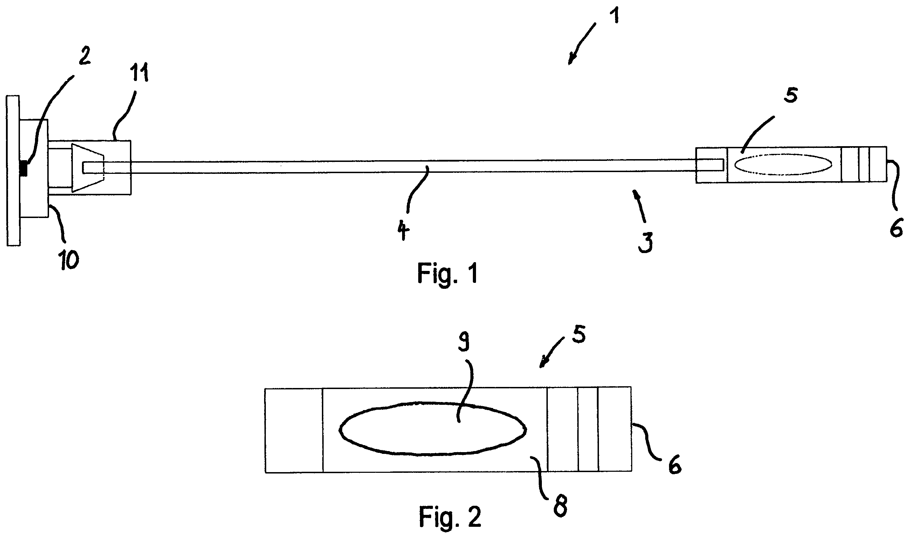

Fig. 1 zeigt eine erfindungsgemäße Drucksensoreinrichtung, insgesamt mit 1 bezeichnet. Die Drucksensoreinrichtung 1 umfasst wenigstens einen Druckmesswandler 2 und einer implantierbaren Sonde 3, wobei die Sonde 3 proximal mit dem wenigstens einen Druckmesswandler 2 verbunden ist. Die Sonde 3 umfasst einen Katheterabschnitt 4 und eine Messspitze 5 am distalen Ende 6 der Sonde 3. Die Sonde 3 hat eine Längserstreckung entlang des Katheterabschnitts 4.

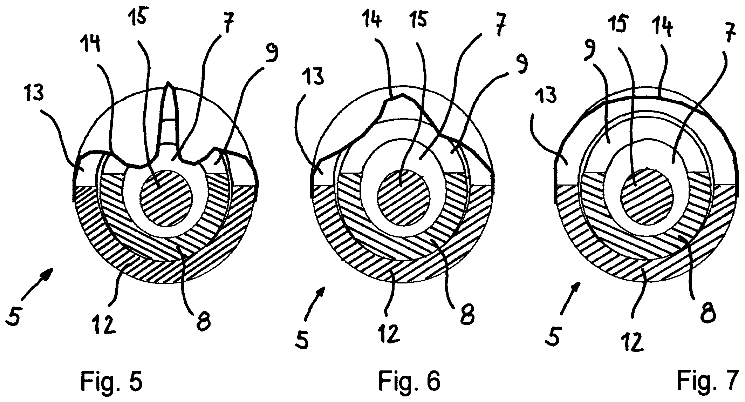

Der Katheterabschnitt 4 weist wenigstens ein Lumen 7 auf, wie in den Figuren 5 bis 7 zu erkennen ist. Das Lumen 7 dient zur Herstellung einer Fluidverbindung von der Messspitze 5 zu dem Druckmesswandler 2. Dazu ist das Lumen 7 mit einer Übertragungsflüssigkeit gefüllt.

Als besonders vorteilhaft und betriebssicher hat sich bei Versuchen herausgestellt, als Übertragungsflüssigkeit eine in Wasser unlösliche perfluorierte Flüssigkeit zu verwenden, die einen Siedepunkt bei Normaldruck von wenigstens 150°C, vorzugsweise von etwa 165°C, aufweist und vollständig verdunstbar ist. Eine solche Übertragungsflüssigkeit reagiert praktisch nicht mit anderen Materialien der Drucksensoreinrichtung 1, gast bei den in vivo vorkommenden Körpertemperaturen nicht aus und verhält sich selbst bei einer Beschädigung während der Handhabung inert und minimiert damit die Gefährdung von Pflegepersonal und Patienten. Eine solche Übertragungsflüssigkeit ist verfügbar mit einer kinematischen Viskosität von etwa 2 mm²/s bis 2,2 mm²/s bei 25°C, z.B. auch 2,1 mm²/s, und weist einen Ausdehnungskoeffizienten von etwa 0,0012 K⁻¹ und eine Oberflächenspannung von etwa 16 mN/m auf (+- 1 mN/m). Mit einer solchen Übertragungsflüssigkeit wurde bei Tests ein gutes Druckübertragungsverhalten erzielt.

Die Messspitze 5 umfasst einen rohrförmigen Abschnitt in Form eines dünnen Metallröhrchens 8, wie in Fig. 2 gezeigt. Das Metallröhrchen 8 ist dabei zweckmäßig aus einem medizinischen rostfreien Stahl oder einer Titanlegierung gefertigt und gegenüber den in einem menschlichen oder tierischen Körper üblicherweise vorkommenden Drücken dimensionsstabil. Das Metallröhrchen 8 weist eine lateral angeordnete Öffnung 9 auf. Die Öffnung 9 weist in Richtung der Längserstreckung eine größere Ausdehnung auf als in Umfangsrichtung. Die vorzugsweise ovale Öffnung 9 weist vorzugsweise ein Verhältnis der Ausdehnung in Richtung der Längserstreckung zur Ausdehnung in Umfangsrichtung von wenigstens 5:1 auf, vorzugsweise auch größer (6:1, 7:1, 8:1, 9:1, 10:1).

Der Katheterabschnitt 4 ist mit einem Gehäuse 10 des Druckmesswandlers 2 in an sich bekannter und geeigneter Weise dicht verbunden, z.B. durch einen UVaushärtbaren Kleber. Die Verbindungsstelle zwischen Katheterabschnitt 4 und Gehäuse 10 des Druckmesswandlers 2 ist zweckmäßig mit einem Stützschlauch 11 gegen Beschädigung geschützt.

Als Material für den Katheterabschnitt 4 hat sich ein TPE-A aus einem Polyether-Block-Amid-Block-Copolymer bewährt. Der Katheterabschnitt 4 ist distal mit dem Metallröhrchen 8 verbunden, beispielsweise mit einem geeigneten Kleber eingeklebt.

Das Metallröhrchen 8 ist wenigstens radial mit einem elastischen Schlauch 12 überzogen aus einem physiologisch unbedenklichen elastischen Kuntststoff, z.B. aus einem Polyurethan. Der Schlauch 12 weist ebenfalls eine Öffnung 13 auf, die in Lage und Form der Öffnung 9 in dem Metallröhrchen 8 entspricht. Für eine einfache Montage hat es sich als zweckmäßig gezeigt, wenn die Öffnung 13 in dem Schlauch 12 geringfügig größer ist, als die Öffnung 9 in dem Metallröhrchen 8. Dadurch lässt sich der Schlauch 12 am Rand seiner Öffnung 13 z.B. einfach mit dem Metallröhrchen 8 verkleben, so dass der Schlauch 12 gegen Verrutschen gesichert ist.

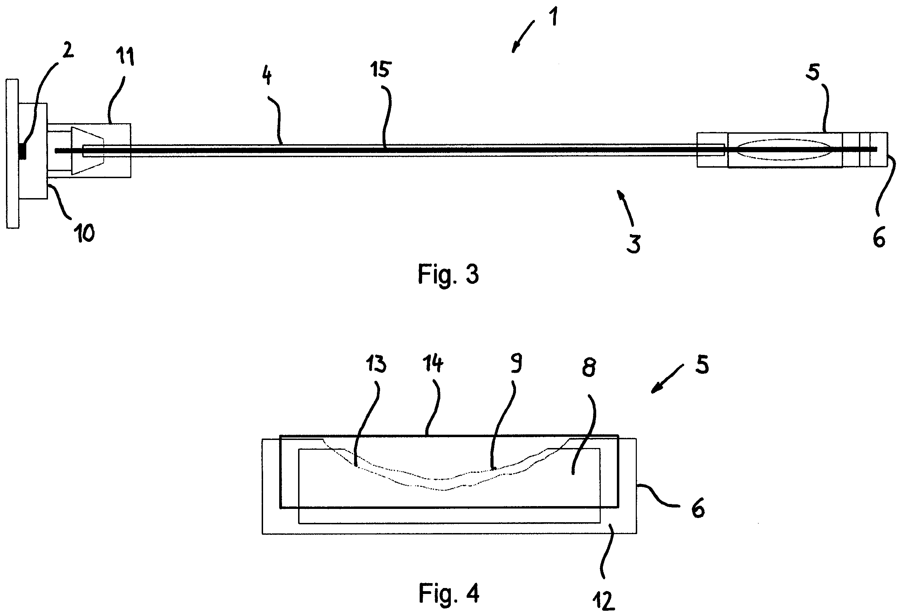

Die Öffnungen 13, 9 in Schlauch 12 und Metallröhrchen 8 sind durch eine elastische Membran 14 abgedeckt. Fig. 4 zeigt schematisch in einer Art Röntgendarstellung die Positionierung von Metallröhrchen 8, Schlauch 12, Membran 14 und den Öffnungen 9 und 13 zueinander in Ansicht von der Seite. Die Membran 14 besteht aus einem gegenüber dem Schlauch 12 wesentlich dünneren Material, das mit dem Schlauch 12 durch Lösungsmittelklebung hermetisch dicht verbunden ist, wie in den Schnittdarstellungen in den Figuren 5 bis 7 zu sehen ist. Als Material für die Membran 14 hat sich ebenfalls ein Polyurethan als sehr geeignet gezeigt. Die Dicke der Membran 14 sollte nicht mehr als 20 µm, vorzugsweise 15µm oder weniger, betragen.

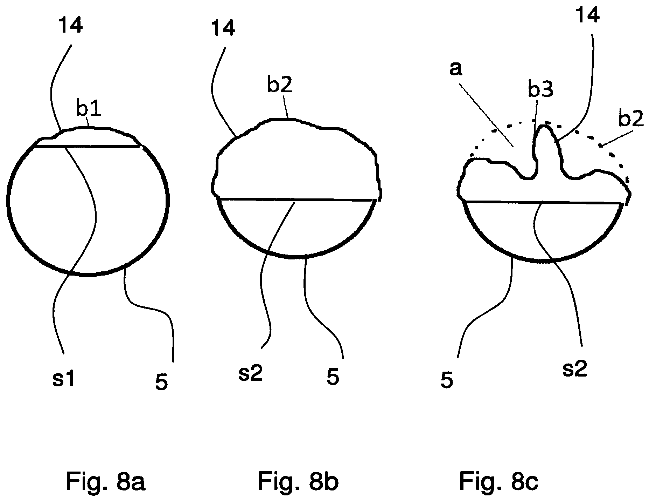

Die wenigstens eine Öffnung 9 in dem rohrförmigen Abschnitt 8 weist in einer Richtung quer zu der Längserstreckung einen von der Membran 14 abgedeckten Umfangsbogenabschnitt b2 auf, der mit der Kontur der Messspitze 5 quer zur Längserstreckung im Wesentlichen fluchtet, wie in den Fig. 7 und 8b zu sehen ist. Ein Sehnenabschnitt s2, der die einander gegenüberliegenden Ränder der Öffnung 9, 13 verbindet, ergibt sich in einer Richtung quer zu der Längserstreckung des rohrförmigen Abschnitts 8, siehe Figuren 8b und 8c. Bei einem Verhältnis der Länge des Umfangsbogenabschnitts b2 zur Länge der Sehne s2 zwischen 1,33 und 1,67, bevorzugt zwischen 1,5 und 1,6, besonders bevorzugt etwa 1,57, kann sich die Membran besonders stark verformen, ohne dass eine Zugspannung in der Membran entsteht. Eine solche Zugspannung würde einen Teil der Druckkräfte aus der Umgebung in das Metallröhrchen 8 leiten und damit das Messergebnis verfälschen. Bei der erfindungsgemäßen Ausgestaltung und erfindungsgemäßer Herstellung steht ein erheblicher Spielraum für durch Temperaturänderungen verursachte Volumenänderungen der Übertragungsflüssigkeit zur Verfügung. Aus dem Vergleich der Darstellungen in den Figuren 8 b und 8c wird dies besonders deutlich. Die Länge des Sehnenabschnittes s2 ist in beiden Darstellungen gleich, ebenso die Längen der Umfangsbogenabschnitte b2 und b3. Der Teil a der Querschnittsfläche quer zu der Längserstreckung des rohrförmigen Abschnitts 8 gibt einen Hinweis auf die Volumenänderung an Übertragungsflüssigkeit durch Temperaturänderung, die bei einer erfindendungsgemäßen Drucksensoreinrichtung 1 praktisch ohne Auswirkung auf die Messgenauigkeit oder das Übertragungsverhalten der Drucksensoreinrichtung 1 bleibt.

Fig. 8a zeigt zum Vergleich eine Darstellung einer Öffnung 9 in dem rohrförmigen Abschnitt 8 mit einem deutlich kleineren Verhältnis der Länge des Umfangsbogenabschnitts b1 zur Länge der Sehne s1.

Bei der Herstellung einer erfindungsgemäßen Drucksensoreinrichtung 1 wird zweckmäßig zunächst zumindest der Katheterabschnitt 4 und die Übertragungsflüssigkeit auf eine vorbestimmte Temperatur oberhalb der für die Drucksensoreinrichtung 1 vorgesehenen Einsatztemperatur gebracht, vorzugsweise etwa 1 K bis etwa 5 K höher als die vorgesehene Einsatztemperatur. Für die Anwendung beim Menschen liegt die vorbestimmte Temperatur vorzugsweise bei etwa 40°C bis 46°C, insbesondere um 45°C. Dann wird das Lumen 7 und die Messpitze 5 mit der temperierten Übertragungsflüssigkeit, wie in den Figuren 5 und 6 zu erkennen ist, so weit gefüllt, dass die Wölbung der Membran 14 quer zur Längserstreckung mit der Kontur der Messspitze 5 quer zur Längserstreckung im Wesentlichen fluchtet, insbesondere nicht über die Kontur der Messspitze 5 quer zur Längserstreckung hinausragt, wie in Fig. 7 zu sehen ist. Anschließend erfolgt blasenfreies Verschließen des Lumens 7. Mit einer nachfolgenden Qualitätskontrolle ist sicherzustellen, dass in der Drucksensoreinrichtung 1 keine Luftblasen gefangen sind, denn diese würden die Druckübertragungseigenschaften dramatisch verschlechtern und die Drucksensoreinrichtung 1 unbrauchbar machen.

Die Füllmenge der Übertragungsflüssigkeit ist also so bestimmt, dass bei einer vorbestimmten Temperatur der Übertragungsflüssigkeit die Wölbung der Membran 14 quer zur Längserstreckung mit der Kontur der Messspitze 5 quer zur Längserstreckung im Wesentlichen fluchtet.

Wie in Fig. 3 gezeigt ist, ist bei einer weiteren bevorzugten Ausführungsform in dem Lumen 7, das die Übertragungsflüssigkeit enthält, eine flexible Seele 15 eingebettet. Dadurch wird die Gefahr eines Knickens des Katheterabschnitts 4 verringert, was zu einem Abquetschen des Lumens 7 und damit zu einem Ausfall der Druckübertragung auf den wenigstens einen Druckmesswandler 2 führen könnte. Auch bei einer übermäßigen Biegung des Katheterabschnittes 4 werden die Wandungen des Lumens 7 durch die Seele 15 auf Abstand gehalten, so dass stets eine ausreichender Querschnittsfläche für die Druckübertragung durch die Übertragungsflüssigkeit offengehalten wird. Durch die Seele 15 in dem Lumen 7 wird ferner die Menge an Überragungsflüssigkeit verringert, was nicht nur die Herstellungskosten für eine erfindungsgemäße Drucksensoreinrichtung 1 vermindert, sondern auch den Einfluss der thermischen Ausdehnung der Übertragungsflüssigkeit auf die Messgenauigkeit.

Bevorzugt besteht die Seele 15 aus einem Polyamid, vorzugsweise aus einem Polyamid 11 oder einem Polyamid 12. Eine Seele aus Polyamid 12 (PA12) bietet den besonderen Vorteil, dass eine erfindungsgemäße Druckmesseinrichtung 1 auch bei operativen Eingriffen unter laufender Röntgenkontrolle gut eingesetzt werden kann, da bei einer solchen Ausführungsform die Seele 15 gegen Röntgenstrahlung beständig ist. Weiter bietet eine Seele aus Polyamid 12 besondere Knickfestigkeit des Katheterabschnitts 4 der Sonde 3 und damit eine besonders hohe Betriebssicherheit der erfindungsgemäßen Druckmesseinrichtung 1 bei Einsatz unter beengten Platzverhältnissen, z.B. in einem Rettungswagen, einem Ambulanzflugzeug, einem mobilen Krankenhaus oder einer mobilen Sanitätseinrichtung. Eine Seele aus Polyamid 11 (PA 11) bietet den Vorzug der Anwendbarkeit gängiger Sterilisierverfahren, ist autoklavierbar, kann mit Ethylenoxid chemisch und mittels Gamma-Strahlung strahlensterilisiert werden. Auch eine Seele aus Polyamid 11 bietet eine hohen Knickschutz für den Katheterabschnitt 4 der Sonde 3. Darüber hinaus ist eine Seele aus einem Polyamid 11 als physiologisch unbedenklich einzustufen.

Wirtschaftlich besonders gut umsetzen lässt sich die Erfindung mit einem Messsystem enthaltend wenigstens eine solche Drucksensoreinrichtung. Dabei ist es besonders vorteilhaft, dass der Katheterabschnitt 4 der Sonde 3 mit der Messspitze 5 über einen Port mit nur geringfügig größerem Innendurchmesser in einen menschlichen oder tierischen Körper implantiert, z.B. in ein Blutgefäß eingeführt werden kann. Dabei kann der den Druckmesswandler 2 mit zugehöriger Elektronik enthaltende Teil der Drucksensoreinrichtung 1 außerhalb des Körpers verbleiben. Durch eine solche Anordnung können die Vorzüge einer direkten Blutdruckmessung mit den geringeren regulatorischen Anforderungen eines passiven Implantats wirtschaftlich vorteilhaft verbunden werden. Darüber hinaus ermöglicht diese einen in vivo Einsatz der Drucksensoreinrichtung 1 auch bei gleichzeitiger Verwendung minimalinvasiver Techniken mit einem erhöhten elektromagnetischen Störpotential, wie z.B. einem Ablationskatheter.

## Patentansprüche

1. Drucksensoreinrichtung (1) für eine medizinische in vivo Anwendung mit wenigstens einem Druckmesswandler (2) und einer implantierbaren Sonde (3), wobei die Sonde (3) proximal mit dem wenigstens einen Druckmesswandler (2) verbunden ist, die Sonde (3) ferner einen Katheterabschnitt (4) und eine Messspitze (5) am distalen Ende (6) der Sonde (3) umfasst, wobei die Sonde (3) eine Längserstreckung entlang des Katheterabschnitts (4) hat,
wobei der Katheterabschnitt (4) wenigstens ein Lumen (7) aufweist zur Herstellung einer Fluidverbindung von der Messspitze (5) zu dem Druckmesswandler (2) und das Lumen (7) mit einer Übertragungsflüssigkeit gefüllt ist,
wobei die Messspitze (5) einen rohrförmigen Abschnitt (8) umfasst mit wenigstens einer lateral angeordneten Öffnung (9), wobei die wenigstens eine Öffnung (9) durch eine elastische Membran (14) abgedeckt ist, und
wobei die Füllmenge der Übertragungsflüssigkeit so bestimmt ist, dass bei einer vorbestimmten Temperatur der Übertragungsflüssigkeit die Wölbung der Membran (14) quer zur Längserstreckung mit der Kontur der Messspitze (5) quer zur Längserstreckung im Wesentlichen fluchtet, **dadurch gekennzeichnet, dass**
der rohrförmige Abschnitt (8) der Messspitze (5) von einem elastischen Schlauch (12) umhüllt ist und die Membran (14) aus einem ähnlichen Material besteht wie der Schlauch (12), und der elastische Schlauch (12) eine Öffnung (13) aufweist, die die Öffnung (9) in dem rohrförmigen Abschnitt (8) überdeckt und die Membran (14) dicht mit dem Schlauch (12) verbunden ist.

2. Drucksensoreinrichtung (1) nach Anspruch 1, **dadurch gekennzeichnet, dass** die Membran (14) und der Schlauch (12) aus einem Polyurethan besteht.

3. Drucksensoreinrichtung (1) nach Anspruch 2, **dadurch gekennzeichnet, dass** die Öffnung (13) in dem Schlauch (12) geringfügig größer ist, als die Öffnung (9) in dem rohrförmigen Abschnitt (8).

4. Drucksensoreinrichtung (1) nach einem der vorhergehenden Ansprüche, **dadurch gekennzeichnet, dass** der rohrförmige Abschnitt aus einem Metallrohr (8) gebildet ist, vorzugsweise aus einem medizinischen rostfreien Stahl oder einer Titanlegierung.

5. Drucksensoreinrichtung (1) nach einem der vorhergehenden Ansprüche, **dadurch gekennzeichnet, dass** die wenigstens eine Öffnung (9) in dem rohrförmigen Abschnitt (8) eine in Richtung der Längserstreckung größere Ausdehnung aufweist, als in Umfangsrichtung, vorzugsweise das Verhältnis der Ausdehnung in Richtung der Längserstreckung zur Ausdehnung in Umfangsrichtung wenigstens 5:1 beträgt, insbesondere etwa 10:1.

6. Drucksensoreinrichtung (1) nach einem der vorhergehenden Ansprüche, **dadurch gekennzeichnet, dass** die wenigstens eine Öffnung (9) in dem rohrförmigen Abschnitt (8) in einer Richtung quer zu der Längserstreckung einen von der Membran (14) abgedeckten Umfangsbogenabschnitt (b2) aufweist, der mit der Kontur der Messspitze (5) quer zur Längserstreckung im Wesentlichen fluchtet, und einen Sehnenabschnitt (s2), der die einander gegenüberliegenden Ränder der Öffnung (9,13) verbindet, wobei das Verhältnis der Länge des Umfangsbogenabschnitts (b2) zur Länge der Sehne (s2) zwischen 1,33 und 1,67, bevorzugt zwischen 1,5 und 1,6, besonders bevorzugt etwa 1,57 beträgt.

7. Drucksensoreinrichtung (1) nach einem der vorhergehenden Ansprüche, **dadurch gekennzeichnet, dass** die Übertragungsflüssigkeit eine in Wasser unlösliche perfluorierte Flüssigkeit umfasst, wobei die perfluorierte Flüssigkeit einen Siedepunkt bei Normaldruck von wenigstens 150°C, vorzugsweise von etwa 165°C, aufweist und vollständig verdunstbar ist.

8. Drucksensoreinrichtung (1) nach einem der vorhergehenden Ansprüche, **dadurch gekennzeichnet, dass** die Übertragungsflüssigkeit eine kinematische Viskosität von etwa 2 mm²/s bis 2,2 mm²/s bei 25°C und/oder einen Ausdehnungskoeffizienten von 0,0012 K⁻¹ und/oder eine Oberflächenspannung von etwa 16 mN/m aufweist.

9. Drucksensoreinrichtung (1) nach einem der vorhergehenden Ansprüche, **dadurch gekennzeichnet, dass** in dem Lumen (7), das die Übertragungsflüssigkeit enthält, eine flexible Seele (15) eingebettet ist.

10. Drucksensoreinrichtung (1) nach Anspruch 9, **dadurch gekennzeichnet, dass** die Seele (15) aus einem Polyamid besteht, vorzugsweise aus einem Polyamid 11 oder einem Polyamid 12.

11. Drucksensoreinrichtung (1) nach einem der vorhergehenden Ansprüche, **dadurch gekennzeichnet, dass** die vorbestimmte Temperatur oberhalb des Temperaturbereiches liegt, bei dem die Drucksensoreinrichtung (1) eingesetzt werden soll, vorzugsweise etwa 1 K bis etwa 5 K höher.

12. Drucksensoreinrichtung (1) nach einem der vorhergehenden Ansprüche, **dadurch gekennzeichnet, dass** die vorbestimmte Temperatur etwa 40°C bis 46°C beträgt, insbesondere um 45°C.

13. Messsystem enthaltend wenigstens eine Drucksensoreinrichtung (1) nach einem der vorhergehenden Ansprüche.

14. Verfahren zur Herstellung einer Drucksensoreinrichtung (1) für eine medizinische in vivo Anwendung mit einer implantierbaren Sonde (3), wobei die Sonde (3) einen Katheterabschnitt (4) und eine Messspitze (5) am distalen Ende (6) der Sonde (3) umfasst,
wobei die Sonde (3) eine Längserstreckung entlang des Katheterabschnitts (4) hat, wobei der Katheterabschnitt (4) wenigstens ein Lumen (7) aufweist zur Aufnahme einer Übertragungsflüssigkeit,
wobei die Messspitze (5) einen rohrförmigen Abschnitt (8) umfasst mit wenigstens einer lateral angeordneten Öffnung (9), wobei die wenigstens eine Öffnung (9) durch eine elastische Membran (14) abgedeckt ist,
**gekennzeichnet durch** die folgenden Schritte:
Temperieren der Sonde (3) und der Übertragungsflüssigkeit auf eine vorbestimmte Temperatur oberhalb der für die Drucksensoreinrichtung (1) vorgesehenen Einsatztemperatur, vorzugsweise etwa 1 K bis etwa 5 K höher als die vorgesehene Einsatztemperatur, insbesondere auf etwa 40°C bis 46°C, insbesondere um 45°C,
Befüllen des Lumens und der Messpitze der temperierten Sonde (3) mit der temperierten Übertragungsflüssigkeit so weit, dass die Wölbung der Membran quer zur Längserstreckung mit der Kontur der Messspitze quer zur Längserstreckung im Wesentlichen fluchtet, insbesondere nicht über die Kontur der Messspitze quer zur Längserstreckung hinausragt und blasenfreies Verschließen des Lumens (7).

## Claims

1. A pressure-sensor device (1) for a medical in-vivo application, with at least one pressure-measurement transformer (2) and with an implantable probe (3), the probe (3) being connected proximally to the at least one pressure-measurement transformer (2), the probe (3) further including a catheter portion (4) and a measuring tip (5) at the distal end (6) of the probe (3), the probe (3) having a longitudinal extent along the catheter portion (4),
wherein the catheter portion (4) exhibits at least one lumen (7) for establishing a fluid connection from the measuring tip (5) to the pressure-measurement transformer (2), and the lumen (7) has been filled with a transmission liquid, wherein the measuring tip (5) includes a tubular portion (8) with at least one laterally arranged opening (9), the at least one opening (9) being covered by an elastic membrane (14), and the filling quantity of the transmission liquid having been determined in such a way that at a predetermined temperature of the transmission liquid the curvature of the membrane (14) at right angles to the longitudinal extent is substantially in alignment with the contour of the measuring tip (5) at right angles to the longitudinal extent, **characterized in that**
the tubular portion (8) of the measuring tip (5) is encased by an elastic hose (12), and the membrane (14) consists of a material similar to that of the hose (12), and the elastic hose (12) exhibits an opening (13) which overlaps the opening (9) in the tubular portion (8), and the membrane (14) has been tightly connected to the hose (12).

2. The pressure-sensor device (1) according to claim 1, **characterized in that** and the membrane (14), and the hose (12), consist of a polyurethane.

3. The pressure-sensor device (1) according to claim 2, **characterized in that** the opening (13) in the hose (12) being slightly larger than the opening (9) in the tubular portion (8).

4. The pressure-sensor device (1) according to any one of the preceding claims, **characterized in that** the tubular portion has been formed from a metal tube (8), preferentially from a medical stainless steel or a titanium alloy.

5. The pressure-sensor device (1) according to any one of the preceding claims, **characterized in that** the at least one opening (9) in the tubular portion (8) has a larger dimension in the direction of longitudinal extent than in the circumferential direction, preferentially the ratio of the dimension in the direction of longitudinal extent to the dimension in the circumferential direction amounts to at least 5:1, in particular about 10:1.

6. The pressure-sensor device (1) according to any one of the preceding claims, **characterized in that** the at least one opening (9) in the tubular portion (8) exhibits, in a direction at right angles to the longitudinal extent, a circumferential-arc portion (b2) covered by the membrane (14), which is substantially in alignment with the contour of the measuring tip (5) at right angles to the longitudinal extent, and a chord portion (s2) which connects the opposing edges of the opening (9, 13), the ratio of the length of the circumferential-arc portion (b2) to the length of the chord (s2) amounting to between 1.33 and 1.67, preferably between 1.5 and 1.6, particularly preferably about 1.57.

7. The pressure-sensor device (1) according to any one of the preceding claims, **characterized in that** the transmission liquid comprises a water-insoluble perfluorinated liquid, the perfluorinated liquid having a boiling-point at normal pressure of at least 150 °C, preferentially of about 165 °C, and being completely evaporable.

8. The pressure-sensor device (1) according to any one of the preceding claims, **characterized in that** the transmission liquid has a kinematic viscosity from about 2 mm²/s to 2.2 mm²/s at 25 °C and/or a coefficient of expansion of 0.0012 K⁻¹ and/or a surface tension of about 16 mN/m.

9. The pressure-sensor device (1) according to any one of the preceding claims, **characterized in that** a flexible core (15) has been embedded in the lumen (7) which contains the transmission liquid.

10. The pressure-sensor device (1) according to claim 9, **characterized in that** the core (15) consists of a polyamide, preferentially of a polyamide 11 or a polyamide 12.

11. The pressure-sensor device (1) according to any one of the preceding claims, **characterized in that** the predetermined temperature lies above the temperature range within which the pressure-sensor device (1) is to be employed, preferentially about 1 K to about 5 K higher.

12. The pressure-sensor device (1) according to any one of the preceding claims, **characterized in that** the predetermined temperature amounts to about 40 °C to 46 °C, in particular around 45 °C.

13. A measuring system comprising at least one pressure-sensor device (1) according to any one of the preceding claims.

14. A method for producing a pressure-sensor device (1) for a medical in-vivo application, with an implantable probe (3),
wherein the probe (3) comprises a catheter portion (4) and a measuring tip (5) at the distal end (6) of the probe (3),
wherein the probe (3) has a longitudinal extent along the catheter portion (4), the catheter portion (4) exhibiting at least one lumen (7) for receiving a transmission liquid,
wherein the measuring tip (5) includes a tubular portion (8) with at least one laterally arranged opening (9), the at least one opening (9) being covered by an elastic membrane (14),
**characterized by** the following steps:
controlling the temperature of the probe (3) and of the transmission liquid to a predetermined temperature above the operating temperature specified for the pressure-sensor device (1), preferentially about 1 K to about 5 K higher than the specified operating temperature, in particular to about 40 °C to 46 °C, in particular around 45 °C,
filling the lumen and the measuring tip of the temperature controlled probe (3) with the temperature-controlled transmission liquid so far that the curvature of the membrane at right angles to the longitudinal extent is substantially in alignment with the contour of the measuring tip at right angles to the longitudinal extent, in particular does not protrude beyond the contour of the measuring tip at right angles to the longitudinal extent, and bubble-free sealing of the lumen (7).

## Revendications

1. Dispositif de détection de pression (1) pour une application médicale in vivo avec au moins un convertisseur de mesure de pression (2) et avec une sonde (3) implantable, dans lequel la sonde (3) est connectée proximalement à l'au moins un convertisseur de mesure de pression (2), la sonde (3) comprend en outre une section de cathéter (4) et une pointe de mesure (5) sur l'extrémité distale (6) de la sonde (3), dans lequel la sonde (3) présente une extension longitudinale le long de la section de cathéter (4),
dans lequel la section de cathéter (4) présente au moins une lumière (7) pour établir une communication fluidique entre la pointe de mesure (5) et le convertisseur de mesure de pression (2) et la lumière (7) est remplie d'un liquide de transmission,
dans lequel la pointe de mesure (5) comprend une section tubulaire (8) avec au moins une ouverture (9) disposée latéralement, dans lequel l'au moins une ouverture (9) est recouverte par une membrane élastique (14), et
dans lequel la quantité de remplissage du liquide de transmission est définie de telle sorte qu'à une température prédéfinie du liquide de transmission, le bombement de la membrane (14) est sensiblement aligné transversalement à l'extension longitudinale avec le contour de la pointe de mesure (5) transversalement à l'extension longitudinale, **caractérisé en ce que**
la section tubulaire (8) de la pointe de mesure (5) est entourée d'un tuyau flexible (12) élastique et la membrane (14) est constituée d'un matériau similaire à celui du tuyau flexible (12), et le tuyau flexible (12) élastique présente une ouverture (13) qui recouvre l'ouverture (9) dans la section tubulaire (8) et la membrane (14) est reliée de manière étanche au tuyau flexible (12).

2. Dispositif de détection de pression (1) selon la revendication 1, **caractérisé en ce que** la membrane (14) et le tuyau flexible (12) sont constitués d'un polyuréthane.

3. Dispositif de détection de pression (1) selon la revendication 2, **caractérisé en ce que** l'ouverture (13) dans le tuyau flexible (12) est légèrement plus grande que l'ouverture (9) dans la section tubulaire (8).

4. Dispositif de détection de pression (1) selon l'une quelconque des revendications précédentes, **caractérisé en ce que** la section tubulaire est formée à partir d'un tube métallique (8), de préférence en un acier inoxydable médical ou en un alliage de titane.

5. Dispositif de détection de pression (1) selon l'une quelconque des revendications précédentes, **caractérisé en ce que** l'au moins une ouverture (9) dans la section tubulaire (8) présente une dilatation plus importante en direction de l'extension longitudinale que dans la direction périphérique, de préférence le rapport entre la dilatation en direction de l'extension longitudinale et la dilation dans la direction périphérique est d'au moins 5:1, en particulier d'environ 10:1.

6. Dispositif de détection de pression (1) selon l'une quelconque des revendications précédentes, **caractérisé en ce que** l'au moins une ouverture (9) dans la section tubulaire (8) présente dans une direction transversale à l'extension longitudinale une section d'arc périphérique (b2) recouverte par la membrane (14) qui s'aligne sensiblement avec le contour de la pointe de mesure (5) transversalement à l'extension longitudinale, et une section de corde (s2), qui relie les bords opposés les uns aux autres de l'ouverture (9,13), dans lequel le rapport entre la longueur de la section d'arc périphérique (b2) et la longueur de la corde (s2) est compris entre 1,33 et 1,67, de manière préférée entre 1,5 et 1,6, est de manière particulièrement préférée d'environ 1,57.

7. Dispositif de détection de pression (1) selon l'une quelconque des revendications précédentes, **caractérisé en ce que** le liquide de transmission comprend un liquide perfluoré insoluble dans l'eau, dans lequel le liquide perfluoré présente un point d'ébullition à une pression normale d'au moins 150 °C, de préférence d'environ 165 °C, et peut être évaporé en totalité.

8. Dispositif de détection de pression (1) selon l'une quelconque des revendications précédentes, **caractérisé en ce que** le fluide de transmission présente une viscosité cinématique d'environ 2 mm²/s à 2,2 mm²/s à 25 °C et/ou un coefficient de dilatation de 0,0012 K ⁻¹ et/ou une tension superficielle d'environ 16 mN/m.

9. Dispositif de détection de pression (1) selon l'une quelconque des revendications précédentes, **caractérisé en ce qu'**une âme (15) flexible est intégrée dans la lumière (7) qui contient le liquide de transmission.

10. Dispositif de détection de pression (1) selon la revendication 9, **caractérisé en ce que** l'âme (15) est constituée d'un polyamide, de préférence d'un polyamide 11 ou d'un polyamide 12.

11. Dispositif de détection de pression (1) selon l'une quelconque des revendications précédentes, **caractérisé en ce que** la température prédéfinie est supérieure à la plage de températures dans laquelle le dispositif de détection de pression (1) doit être utilisé, de préférence est plus élevée d'environ 1 K à environ 5 K.

12. Dispositif de détection de pression (1) selon l'une quelconque des revendications précédentes, **caractérisé en ce que** la température prédéfinie est d'environ 40 °C à 46 °C, en particulier de 45 °C.

13. Système de mesure comportant au moins un dispositif de détection de pression (1) selon l'une quelconque des revendications précédentes.

14. Procédé de fabrication d'un dispositif de détection de pression (1) pour une application médicale in vivo avec une sonde (3) implantable, dans lequel la sonde (3) comprend une section de cathéter (4) et une pointe de mesure (5) sur l'extrémité distale (6) de la sonde (3),
dans lequel la sonde (3) présente une extension longitudinale le long de la section de cathéter (4), dans lequel la section de cathéter (4) présente au moins une lumière (7) pour recevoir un liquide de transmission,
dans lequel la pointe de mesure (5) comprend une section tubulaire (8) avec au moins une ouverture (9) disposée latéralement, dans lequel l'au moins une ouverture (9) est recouverte par une membrane élastique (14),
**caractérisé par** les étapes suivantes :
de thermorégulation de la sonde (3) et du liquide de transmission à une température prédéfinie supérieure à la température d'utilisation prévue pour le dispositif de détection de pression (1), de préférence plus élevée d'environ 1 K à environ 5 K que la température d'utilisation prévue, en particulier à environ 40 °C à 46 °C, en particulier à 45 °C,
de remplissage du lumen et de la pointe de mesure de la sonde (3) thermorégulée avec le liquide de transmission thermorégulé de telle sorte que le bombement de la membrane est sensiblement aligné avec le contour de la pointe de mesure transversalement à l'extension longitudinale, en particulier ne dépasse pas du contour de la pointe de mesure transversalement à l'extension longitudinale, et de fermeture de la lumière (7) sans bulle.
